(19) Europäisches Patentamt
European Patent Office
Office européen des brevets

(11) Publication number: **0 187 739**
**B1**

(12) # EUROPEAN PATENT SPECIFICATION

(45) Date of publication of patent specification: **23.03.88**

(51) Int. Cl.$^4$: **C 08 G 18/77**, C 07 D 495/08

(21) Application number: **84902683.6**

(22) Date of filing: **29.06.84**

(86) International application number:
**PCT/US84/01032**

(87) International publication number:
**WO 86/00318 16.01.86 Gazette 86/02**

(54) **9-THIABICYCLONONANEDIISOCYANATES AND POLYMERS PREPARED THEREFROM.**

(43) Date of publication of application:
**23.07.86 Bulletin 86/30**

(45) Publication of the grant of the patent:
**23.03.88 Bulletin 88/12**

(84) Designated Contracting States:
**DE FR GB NL**

(56) References cited:
**GB-A-1 061 472**
**GB-A-1 061 473**
**US-A-2 729 618**
**US-A-3 248 373**
**US-A-3 644 415**

**J. ORGANIC CHEM., VOL. 31, NO. 6, ISSUED 13 JUNE 1966, COREY ET AL. PP. 1663-1668**

**J. ORGANIC CHEM. VOL. 31, NO. 6, ISSUED 13 JUNE 1966, WEIL ET AL. PP. 1669-1679**

(73) Proprietor: **THE DOW CHEMICAL COMPANY**
**2030 Dow Center Abbott Road P.O. Box 1967**
**Midland, MI 48640 (US)**

(72) Inventor: **HUGHES, David, W.**
**3812 Sharon Road**
**Midland, MI 48640 (US)**
Inventor: **SUTTON, Tonja, R.**
**1801 Brookfield Drive**
**Midland, MI 48640 (US)**

(74) Representative: **Sternagel, Hans-Günther, Dr. et al**
**Patentanwälte Dr. Michael Hann Dr. H.-G. Sternagel Sander Aue 30**
**D-5060 Bergisch Gladbach 2 (DE)**

**EP 0 187 739 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to novel 9-thiabicyclononanediisocyanates which have utility as monomers in making polyurethanes and other polymers.

A polymer is a large molecule built up by the repetition of small, simple chemical units called monomers. The character of the monomer unit has a strong effect on the physical and chemical properties of the polymer. For example, it is common to incorporate a paraphenylene group into a monomer to add rigidity to the polymer chain. This can engender desirable properties in the polymer such as: raising the melting point, increasing the stress/strain property ratios and improving the heat distortion performance.

The incorporation of aromatic nuclei in polymer chains, however, has its drawbacks. Polymers containing aromatic nuclei are susceptible to deterioration. They may stiffen and become brittle, change color, or yellow and weaken. Opaque fillers, light stabilizers and antioxidants are added to alleviate these problems. Aliphatic monomers yield polymers which are less susceptible to degradation but do not impart the same rigidity.

This invention is directed to 9-thiabicyclononanediisocyanates of the structural formula:

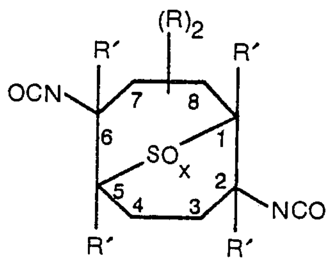

or

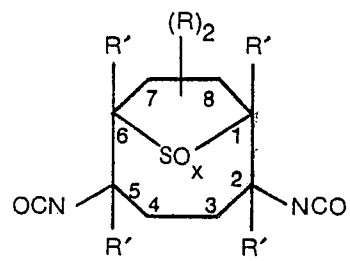

(I)

wherein each R which may be the same or different is an alkyl group containing 1 to 3 carbon atoms; each R′ which may be the same or different is hydrogen or methyl, at least two R′ are hydrogen; and x is 0, 1 or 2. Preferably all R′ are hydrogen. More preferably x is zero. Most preferably each R is methyl.

Incorporation of these monomers into a polyurethane or other polymer chain induces chain rigidity as evidenced by stress/strain properties and phase change properties. It is presumed that the sulfur bridge is responsible for decreasing the degree of freedom in the molecule and thus imparting stiffening to the polymer chain.

Preparation of the thiabicyclononanes starts with $C_4$—$C_8$ di-unsaturated hydrocarbons. Examples of such hydrocarbons include butadiene, piperylene or 1,3-pentadiene; 1,3-hexadiene; 1,3-heptadiene; 5-methyl-1,3-hexadiene; isoprene; and 3-methyl-1,3-pentadiene. The starting dienes may be used singly or a mixture of starting dienes may be used. The starting materials may be represented by the formula:

$$CH_2{=}CH{-}\underset{\displaystyle R'}{\underset{|}{C}}{=}CH{-}R \qquad \text{(II)}$$

wherein R and R′ are as defined above. Examples of R are hydrogen, methyl, ethyl, n-propyl and isopropyl. Preferably R is methyl. R′ is preferably hydrogen. Piperylene is a preferred diene.

The dienes II are cyclodimerized to form a 1,5-cyclooctadiene III.

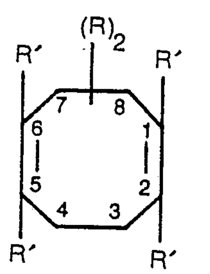

(III)

Cyclodimerization of the diene is a known reaction. Examples of this reaction can be found at J. A. Berson et al., *JACS,* 98 (19), pp. 5937—68 (1976) (Chem. Abstr. 86:70,955q); and U. M. Dzhemilev et al., *Neftekhimiya,* 15 (6), pp. 819—24 (1975) (Chem. Abstr. 84:121,245b).

A preferred method of conducting the cyclodimerization of piperylene is by heating and stirring a mixture of piperylene in other hydrocarbons with a catalyst containing an iron (III) salt, a trialkyl aluminum compound and a chelating nitrogen ligand.

These processes yield varying amounts of the following isomers when the R group is methyl.

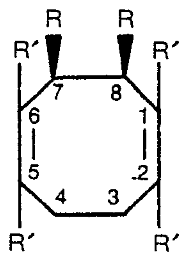

+

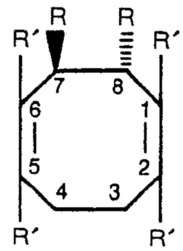

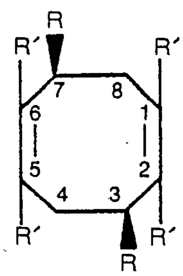

+

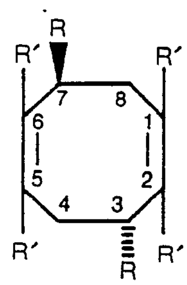

(IIIa)

These isomers as well as isomers where R′ is $CH_3$, are collectively included in the definition of the cyclooctadienes III. Note that the R groups are as defined above and are not attached to the carbons of the double bond. The double bonds are positioned between the 1 and 2 carbons and between the 5 and 6 carbons. The R groups may be attached only to the 3, 4, 7 and 8 carbons. When R′ is methyl, that group is attached to a carbon of the double bond. While the R′ methyl may be attached to carbons 1, 2, 5 or 6, only two R′ may be methyl.

The cyclooctadienes III may be converted to thiabicyclononanes IV by the reaction disclosed in Weil et al. in *J. Org. Chem.*, 31 (6), pp. 1669—1679 (1966); or Corey et al. in *J. Org. Chem.*, 31 (6), pp. 1663—1668 (1966); or Tolstikov et al. in *Zh. Org. Khim.*, 16 (7), pp. 1408—1418 (1980); or British patents 1,061,472 and 1,061,473.

These references teach that a cyclooctadiene III may be converted to the thiabicyclononane dichloride IV by treatment with sulfur dichloride. This yields

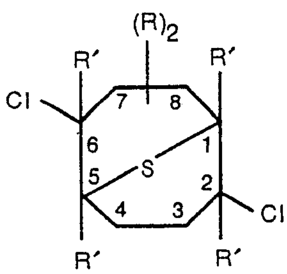

or

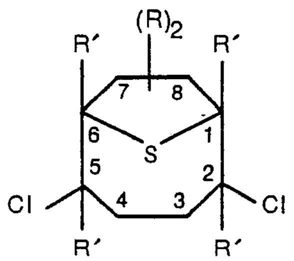

(IV)

where R and R′ are as defined in I and the R groups may be connected to the 3, 4, 7 or 8 ring carbons, but not the carbons directly attached to either the sulfur or chlorine atoms. The chlorine atoms are not attached to vicinal carbon atoms. The term TBCN is used hereinafter to describe generically the 9-thiabicyclononane IV, divalent radical without the chlorine atoms, which may be derived from the generic cyclooctadiene III.

Either or both of the [3.3.1] and [4.2.1] structures are found in the product as it has been found that the two structures are interconvertible during any reaction, even by merely dissolving in an ionizing solvent.

The preparation of the TBCN dichloride IV is accomplished by reacting the corresponding cyclooctadiene III with sulfur dichloride or other sulfur chloride. This is most conveniently done in the liquid phase, although it can also be accomplished in the vapor phase. The reaction is exothermic and, therefore, the reactants should be admixed by slow addition of one to the other, or, preferably, of both to a mutual solvent. Suitable solvents are any that are substantially inert to sulfur dichloride or the sulfur chloride and cyclooctadiene. Illustrative examples of suitable solvents include hydrocarbons such as toluene, benzene, hexane, cyclohexane, mineral spirits, chlorocarbons such as methylene chloride, carbon tetrachloride, ethylene dichloride, trichloroethylene, perchloroethylene, chlorobenzene, ethers such as diethyl ether, or miscellaneous solvents such as carbon disulfide, thionyl chloride, acetic anhydride, acetyl chloride, nitromethane, nitrobenzene, and dimethyl formamide.

The reaction temperature is from −80°C to 150°C, however, the preferred range is between −20°C and 100°C. It is particularly convenient to employ reaction temperatures near ambient temperature, and to cool the reaction by water-jacketing the reactor using water, also at ambient temperature.

The reaction is very rapid and generally is complete within a few second to a few hours after the reactants are admixed, depending on temperature. Therefore, a catalyst is not necessary. Nonetheless, if desired, the reaction may be catalyzed by addition of Lewis acids (e.g., $FeCl_3$), iodine, light of peroxides.

While sulfur dichloride is the preferred reactant, sulfur monochloride may be employed to obtain the TBCN dichlorides; however, the use of sulfur monochloride results in a more complex reaction mixture which entails troublesome purification steps. Sulfur tetrachloride may also be used, with resultant formation of some TBCN having more than two chlorine atoms per molecule.

A preferred method of making the TBCN dichloride is by admitting separate streams of the corresponding cyclooctadiene III and sulfur dichloride, each dissolved in an appropriate solvent into a line containing a static mixer. The concentrations of the feed solutions and the ratio in which they are admitted into the reactor are controlled to ensure a slight molar excess of sulfur dichloride. The line is cooled to ensure a maximum reactor temperature of −5°C. Preferably the sulfur dichloride is stabilized according to the method described in US—A—3,071,441.

The 9-oxides or 9,9-dioxides, i.e., where x is 1 or 2, are prepared by the oxidation of the corresponding sulfide. Prior to oxidation, the chlorine atoms should be replaced by the isocyanate groups as described herein. Illustrative oxidizing agents include hydrogen peroxide, peracetic acid, perbenzoic acid, perphthalic acid or other peroxy organic acids; nitric acid; nitrogen dioxide or tetraoxide; permanganates; chromic acid or dichromates; bromic acid or bromates; hypochlorous acid or hypochlorites; and ozone or molecular oxygen (preferably using a catalyst such as vanadium oxide or nitrogen dioxide).

The TBCN dichlorides may be converted to the TBCN diisocyanate by known means. Exemplary means include treating the TBCN dichloride with a cyanate salt. For example, the TBCN dichloride may be reacted with potassium cyanate in acetonitrile at room temperature. Preferred cyanate salts are sodium, potassium or ammonium cyanate. A phase-transfer catalyst is recommended when using sodium cyanate.

Examples of preferred thiabicyclic diisocyanates are dialkyl-9-thiabicyclononane diisocyanate isomers such as 2 - *endo* - 6 - *endo* - 2,6 - diisocyanato - 4 - *endo* - 8 - *exo* - 4,8 - dimethyl - 9 - thiabicyclo-[3.3.1]nonane; 2 - *endo* - 6 - *endo* - 2,6 - diisocyanato - 4 - *exo* - 8 - *exo* - 4,8 - dimethyl - 9 - thiabicyclo[3.3.1]nonane; 2 - *endo* - 6 - *endo* - 2,6 - diisocyanato - 4 - *endo* - 8 - *endo* - 4,8 - dimethyl - 9 - thiabicyclo[3.3.1]nonane; 2 - *endo* - 6 - *endo* - 2,6 - diisocyanato - 3 - *endo* - 7 - *endo* - 3,7 - dimethyl - 9 - thiabicyclo[3.3.1]nonane; 2 - *endo* - 6 - *endo* - 2,6 - diisocyanato - 3 - *exo* - 7 - *exo* - 3,7 - dimethyl - thiabicyclo[3.3.1]nonane; 2 - *endo* - 6 - *endo* - 2,6 - diisocyanato - 3 - *endo* - 7 - *exo* - 3,7 - dimethyl - 9 - thiabicyclo[3.3.1]nonane; 2 - *endo* - 6 - *endo* - 2,6 - diisocyanato - 3 - *exo* - 4 - *exo* - 3,4 - dimethyl - 9 - thiabicyclo[3.3.1]nonane; 2 - *endo* - 6 - *endo* - 2,6 - diisocyanato - 3 - *endo* - 4 - *exo* - 3,4 - dimethyl - 9 - thiabicyclo[3.3.1]nonane; 2 - *endo* - 6 - *endo* - 2,6 - diisocyanato - 3 - *exo* - 4 - *endo* - 3,4 - dimethyl - 9 - thiabicyclo[3.3.1]nonane; 2 - *endo* - 6 - *endo* - 2,6 - diisocyanato - 3 - *endo* - 4 - *endo* - 3,4 - dimethyl - 9 - thiabicyclo[3.3.1]nonane; 2 - *endo* - 5 - *endo* - 2,5 - diisocyanato - 7 - *endo* - 8 - *endo* - 7,8 - dimethyl - 9 - thiabicyclo[4.2.1]nonane; 2 - *endo* - 6 - *endo* - 2,6 - diisocyanato - 7 - *exo* - 1,7 - dimethyl - 9 - thiabicyclo[3.3.1]nonane; 2 - *endo* - 6 - *endo* - 2,6 - diisocyanato - 7 - *endo* - 1,7 - dimethyl - 9 - thiabicyclo[3.3.1]nonane; 2 - *endo* - 6 - *endo* - 2,6 - diisocyanato - 4 - *exo* - 1,4 - dimethyl - 9 - thiabicyclo[3.3.1]nonane; 2 - *endo* - 6 - *endo* - 2,6 - diisocyanato - 4 - *endo* - 1,4 - dimethyl - 9 - thiabicyclo[3.3.1]nonane; 2 - *endo* - 5 - *endo* - 2,5 - diisocyanato - 7 - *endo* - 1,7 - dimethyl - 9 - thiabicyclo[3.3.1]nonane; with normally liquid mixtures of two or more such isomers being especially preferred.

Other isomers which are desirable include 2 - *endo* - 5 - *endo* - 2,5 - diisocyanato - 7 - *exo* - 8 - *exo* - 7,8 - dimethyl - 9 - thiabicyclo[4.2.1]nonane; 2 - *endo* - 5 - *endo* - 2,5 - diisocyanato - 7 - *endo* - 8 - *exo* - 7,8 - dimethyl - 9 - thiabicyclo[4.2.1]nonane; 2 - *endo* - 5 - *endo* - 2,5 - diisocyanato - 3 - *exo* - 4 - *exo* - 3,4 - dimethyl - 9 - thiabicyclo[4.2.1]nonane; 2 - *endo* - 5 - *endo* - 2,5 - diisocyanato - 3 - *endo* - 4 - *endo* - 3,4 - dimethyl - 9 - thiabicyclo[4.2.1]nonane; 2 - *endo* - 5 - *endo* - 2,5 - diisocyanato - 3 - *endo* - 4 - *exo* - 3,4 - dimethyl - 9 - thiabicyclo[4.2.1]nonane; 2 - *endo* - 5 - *endo* - 2,5 - diisocyanato - 3 - *endo* - 7 - *exo* - 3,7 - dimethyl - 9 - thiabicyclo[4.2.1]nonane; 2 - *endo* - 5 - *endo* - 2,5 - diisocyanato - 3 - *endo* - 7 - *endo* - 3,7 - dimethyl - 9 - thiabicyclo[4.2.1]nonane; 2 - *endo* - 5 - *endo* - 2,5 - diisocyanato - 3 - *endo* - 7 - *exo* - 3,7 - dimethyl - 9 - thiabicyclo[4.2.1]nonane; 2 - *endo* - 5 - *endo* - 2,5 - diisocyanato - 3 - *exo* - 7 - *endo* - 3,7 - dimethyl - 9 - thiabicyclo[4.2.1]nonane; 2 - *endo* - 6 - *endo* - 2,6 - diisocyanato - 2 - *exo* - 7 - *exo* - 2,7 - dimethyl - 9 - thiabicyclo[3.3.1]-nonane; 2 - *endo* - 6 - *endo* - 2,6 - diisocyanato - 2 - *exo* - 7 - *endo* - 2,7 - dimethyl - 9 - thiabicyclo-[3.3.1]nonane; 2 - *endo* - 6 - *endo* - 2,6 - dimethyl - 2 - *exo* - 4 - *exo* - 2,4 - dimethyl - 9 - thiabicyclo-[3.3.1]nonane; 2 - *endo* - 6 - *endo* - 2,6 - diisocyanato - 2 - *exo* - 4 - *endo* - 2,4 - dimethyl - 9 - thiabicyclo[3.3.1]nonane; 2 - *endo* - 5 - *endo* - 2,5 - diisocyanato - 2 - *exo* - 4 - *exo* - 2,4 - dimethyl - 9 - thiabicyclo[4.2.1]nonane; 2 - *endo* - 5 - *endo* - 2,5 - diisocyanato - 2 - *exo* - 4 - *endo* - 2,4 - dimethyl - 9 - thiabicyclo[4.2.1]nonane; 2 - *endo* - 5 - *endo* - 2,5 - diisocyanato - 2 - *exo* - 7 - *exo* - 2,7 - dimethyl - 9 - thiabicyclo[4.2.1]nonane; 2 - *endo* - 5 - *endo* - 2,5 - diisocyanato - 2 - *exo* - 7 - *endo* - 2,7 - dimethyl - 9 - thiabicyclo[4.2.1]nonane; 2 - *endo* - 5 - *endo* - 2,5 - diisocyanato - 4 - *exo* - 1,4 - dimethyl - 9 - thiabicyclo[3.3.1]nonane; 2 - *endo* - 5 - *endo* - 2,5 - diisocyanato - 4 - *endo* - 1,4 -

dimethyl - 9 - thiabicyclo[3.3.1]nonane; and 2 - *endo* - 5 - *endo* - 2,5 - diisocyanato - 7 - *exo* - 1,7 - dimethyl - 9 - thiabicyclo[3.3.1]nonane.

The dimethyl TBCN diisocyanate isomer mixture, where R = $CH_3$, is a liquid at room temperature. This improves the processability of the compound over the unsubstituted TBCN diisocyanate, which melts between 71°C and 72.5°C. The dimethyl TBCN diisocyanate isomer mixture may be pumped into a liquid system whereas the unsubstituted material must be melted or dissolved to be pumped.

The TBCN diisocyanates of the invention may be used to form polyurethanes, polyureas, polyureaurethanes, polyurethane isocyanurates, polyurea isocyanurates and polyisocyanurates by step reaction polymerization.

In polyurethanes, polyureas and polyureaurethanes, the residue of the monomer units of the 9-thiabicyclononanediisocyanates of this invention has the formula:

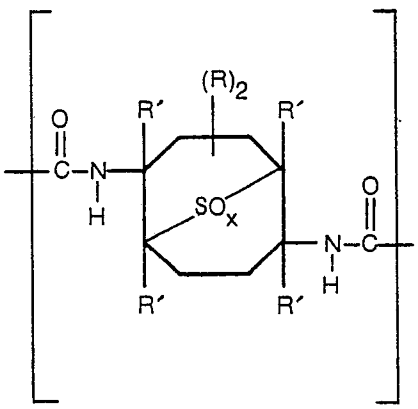

or

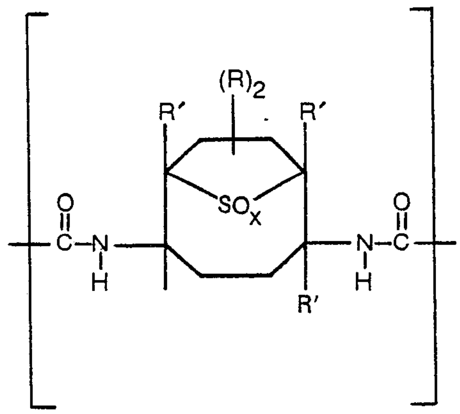

(V)

wherein R, R′, and x have their earliest mentioned definitions and positions.

The polyisocyanurates contain a trifunctional trimer unit which contains three TBCN units. The structure is:

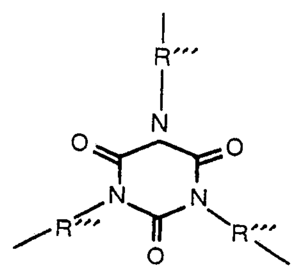

(VI)

wherein R‴ is

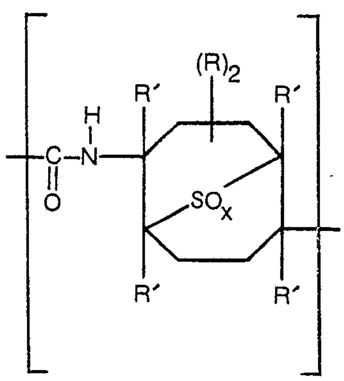

or

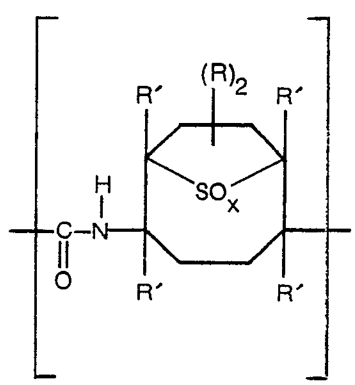

(VIa)

and R, R′, and x have their above-defined meanings.

The polymers of this invention contain a characterizing amount of monomer units of the above formulae. By characterizing amount it is meant a sufficient amount of the residue of the TBCN monomer units V are present in the polymer so that the polymer exhibits properties resulting from the presence of the residue of the TBCN monomer V. Preferably the polymer contains at least 0.5 mole percent of the residue of the monomer units V and more preferably at least 10 mole percent and even more preferably at least 40 mole percent of the residue of the monomer units V.

Polyurethanes, polyureas and polyurea-urethanes of this invention are prepared by reacting a polyahl with the TBCN diisocyanate I in the presence of a urethane and/or a urea catalyst, if required, in proportions such that upon subjecting the formulation to conditions known in the art, a useful article is formed. These polymers may be prepared using high pressure reaction injection molding (RIM) techniques or low pressure mixing techniques known in the art.

The polyahl suitably employed in the practice of this invention includes any organic compound having (1) at least two active hydrogen moieties, and (2) a number average molecular weight ($M_n$) of at least 60. Preferably, the polyahl is a polymer having an $M_n$ of at least 200. For the purposes of this invention, an active hydrogen moiety refers to a moiety containing a hydrogen atom which, because of its position in the molecule, displays significant activity according to the Zerewitnoff test described by Woller in the *Journal of American Chemical Society,* Vol. 49, page 3181 (1927). Illustrative of such active hydrogen moieties are —COOH, —OH, $—NH_2$, —NH—, $—CONH_2$, —SH and —CONH—. Typical polyahls include polyols, polyamines, polyamides, polymercaptans and polyacids.

Of the foregoing polyahls, the polyols are preferred. Examples of such polyols are polyether polyols, polyester polyols, hydroxy functional acrylic polymers, hydroxyl-containing epoxy resins, polyhydroxy terminated polyurethane polymers, polyhydroxyl-containing phosphorus compounds and alkylene oxide adducts of polyhydric thioethers including polythioethers, acetals including polyacetals, aliphatic and aromatic polyols and thiols including polythiols, ammonia and amines including aromatic, aliphatic and heterocyclic amines including polyamines as well as mixtures thereof. Alkylene oxide adducts of compounds which contain two or more different groups within the above-defined classes may also be used such as amino alcohols which contain an amino group and hydroxyl group. Also alkylene oxide adducts of compounds which contain one —SH group and one —OH group, as well as those which contain an amino group and a —SH group, may be used.

Polyether polyols which are most advantageously employed as the polyahl in the practice of this invention are the polyalkylene polyether polyols including the polymerization products of alkylene oxides and other oxiranes with water or polyhydric alcohols having from two to eight hydroxyl groups. Exemplary alcohols that are advantageously employed in making the polyether polyol include ethylene glycol, 1,3-propylene glycol, 1,2-propylene glycol, 1,4-butylene glycol, 1,3-butylene glycol, 1,2-butylene glycol, 1,5-pentane diol, 1,7-heptane diol, glycerin, 1,1,1-trimethylolpropane, 1,1,1-trimethylolethane, hexane-1,2,6-triol, α-methyl glucoside, pentaerythritol, erythritol, pentatols and hexatols. Also included within the term "polyhydric alcohol" are sugars such as glucose, sucrose, fructose and maltose as well as compounds derived from phenols such as 2,2-(4,4'-hydroxyphenyl)propane, commonly known as bisphenol A. Illustrative oxiranes that are advantageously employed in the preparation of the polyether polyol include simple alkylene oxides such as ethylene oxide, propylene oxide, butylene oxide, and amylene oxide; glycidyl ethers such as t-butyl glycidyl ether and phenyl glycidyl ether; and random or block copolymers of two or more of these oxiranes. The polyalkylene polyether polyols may be prepared from other starting materials such as tetrahydrofuran and alkylene oxide-tetrahydrofuran copolymers; epihalohydrins such as epichlorohydrin; as well as aralkylene oxides such as styrene oxide. The polyalkylene polyether polyols may have primary, secondary or tertiary hydroxyl groups and, preferably, are polyethers prepared from alkylene oxides having from two to six carbon atoms such as ethylene oxide, propylene oxide and butylene oxide. The polyalkylene polyether polyols may be prepared by any known process such as, for example, the process disclosed by Wurtz in 1859 and *Encyclopedia of Chemical Technology,* Vol. 7, pp. 257—262, published by Interscience Publishers, Inc. (1951), or in US—A—1,922,459. Suitable polyether polyols and processes for preparing them are also described in Schick, M. J., *Nonionic Surfactants,* Marcel Dekker, Inc., New York (1967), US—A—2,891,073; 3,058,921; 2,871,219 and British Patent 898,306. Polyether polyols which are most preferred include the alkylene oxide addition products of water, trimethylolpropane, glycerine, pentaerythritol, sucrose, sorbitol, propylene glycol and blends thereof having hydroxyl equivalent weights of from 250 to 5000.

Suitable polyhydric thioethers which are sometimes advantageously condensed with alkylene oxides include the reaction product of thiodiglycol with alkylene oxides or dihydric alcohols such as disclosed above.

Polyhydroxyl-containing phosphorus compounds which are optionally used include those compounds disclosed in US—A—3,639,542. Preferred polyhydroxyl-containing phosphorus compounds are prepared from alkylene oxides and acids of phosphorus having a $P_2O_5$ equivalency of from 72 percent to 95 percent.

Polyacetals (acetal resins) which are optionally reacted with alkylene oxides or other oxiranes include the reaction product of formaldehyde or other suitable aldehyde with a polyhydric alcohol or an oxirane such as those disclosed above. Polyacetals derived from acetone or from cyclic acetals are also suitably employed.

Aliphatic and aromatic thiols which are optionally reacted with alkylene oxides and other oxiranes include alkane thiols such as 1,2-ethane dithiol, 1,2-propane dithiol and 1,6-hexane dithiol; alkene thiols such as 2-butene-1,4-dithiol; and alkyne thiols such as 3-hexyne-1,6-dithiol; and arene thiols such as 1,4-benzene dithiol. Other thiols suitable for this purpose are hydrogen sulfide as well as thio functional polymers such as polyvinylbenzyl thiol.

Acids and amides which are optionally reacted with alkylene oxides and other oxiranes include difunctional fatty acids such as hydroxystearic and dihydroxystearic acid as well as amides such as fatty

acid alkanol amides, e.g., lauroyl monoethanolamide; diacids such as adipic and terephthalic acid; sulfonamides and other acids and amides set forth in Schick, *supra*.

Additional polyethers and methods for their preparation are set forth in Schick, *supra*.

Examples of suitable hydroxyl-containing polyesters include those obtained from polycarboxylic acids and polyhydric alcohols. Examples of suitable polycarboxylic acids include oxalic acid, malonic acid, succinic acid, glutaric acid, adipic acid, pimelic acid, suberic acid, azelaic acid, sebacic acid, brassylic acid, thapsic acid, maleic acid, fumaric acid, glutaconic acid, α-hydromuconic acid, β-hydromuconic acid, α-butyl-α-ethyl-glutaric acid, α,β-diethylsuccinic acid, isophthalic acid, terephthalic acid, hemimellitic acid, and 1,4-cyclohexane-dicarboxylic acid. Any suitable polyhydric alcohol including both aliphatic and aromatic may be used such as ethylene glycol, 1,3-propylene glycol, 1,2-propylene glycol, 1,4-butylene glycol, 1,3-butylene glycol, 1,2-butylene glycol, 1,5-pentane diol, 1,4-pentane diol, 1,3-pentane diol, 1,6-hexane diol, 1,7-heptane diol, glycerol, 1,1,1-trimethylolpropane, 1,1,1-trimethylolethane, hexane-1,2,6-triol, α-methyl glucoside, pentaerythritol and sorbitol. Also included with the term "polyhydric alcohol" are compounds derived from phenols such as 2,2-(4,4'-hydroxyphenyl)propane, commonly known as bisphenol A, bis(4,4'-hydroxyphenyl)sulfide and bis-(4,4'-hydroxyphenyl)sulfone.

Of particular interest in forming the polyurea-urethanes of the present invention are the partially aminated and/or partially amine terminated polyethers as described in US—A—4,269,945 and EP—A—93,861; 93,862 and 81,701. To prepare polyureas, it is desirable to use totally aminated and/or totally amine terminated polyols obtained by the process described in US—A—4,153,581.

Amines which are optionally reacted with alkylene oxides and other oxiranes include aromatic amines such as aniline, o-chloroaniline, p-amino aniline, 1,5-diamino naphthalene, methylene dianiline, the condensation products of aniline and formaldehyde and 2,4-diamino toluene; aliphatic amines such as methylamine, triisopropanolamine, isopropanolamine, diisopropanolamine, ethylenediamine, 1,3-propylenediamine, 1,4-butylenediamine and 1,3-butylenediamine, and mixtures thereof.

Other polyahls suitably employed include polylactones; hydroxy functional acrylic polymers such as polymers of hydroxyethyl acrylate and hydroxypropyl acrylate; polyvinyl acetate and other polymers of vinyl acetate and other ethylenically unsaturated carboxylic acids; hydroxyl-containing epoxy resins; ureaformaldehyde and melamine-formaldehyde resins; hydroxyl-containing polycarbonates and polyurethanes; methylol resins; starches and other cellulosic polymers; esters of phosphoric, sulfonic, sulfuric and boric acid; and polypeptides.

In addition, the polyahl suitably contains a copolymer dispersed therein (often such dispersions are called copolymer polyols), e.g., copolymer polyols described in US—B—29118, US—B—29014 and US—A—4,390,645.

In addition to the preferred polyether polyols and partially and totally amine terminated polyether polyols, polyalkyl thiapolycyclic polyahls, especially dimethyl 9-thiobicyclononanediamines are also preferably employed herein as the polyahl or as a chain extender used in combination with the aforementioned polyahls.

The polyisocyanates which may be employed in combination with the thiapolycyclic polyisocyanates include aromatic, aliphatic and cycloaliphatic polyisocyanates and combinations thereof. Representative of these types are the diisocyanates such as m-phenylene diisocyanate, tolylene-2,4-diisocyanate, tolylene-2,6-diisocyanate, hexamethylene-1,6-diisocyanate, tetramethylene-1,4-diisocyanate, cyclohexane-1,4-diisocyanate, hexahydrotolylene diisocyanate (and isomers), naphthylene-1,5-diisocyanate, 1-methoxy-phenyl-2,4-diisocyanate, diphenylmethane-4,4-diisocyanate, 4,4'-biphenylene diisocyanate, 3,3'-dimethoxy-4,4'-biphenyl diisocyanate, 3,3'-dimethyl-4,4'-biphenyl diisocyanate, and 3,3'-dimethyl-diphenylmethane-4,4'-diisocyanate; the triisocyanates such as 4,4',4'-triphenylmethane triisocyanate, polymethylene polyphenylisocyanate and tolylene-2,4,6-triisocyanate; and the tetraisocyanates such as 4,4'-dimethyldiphenylmethane-2,2',5,5'-tetraisocyanate. Especially useful due to their availability and properties are tolylene diisocyanate, diphenylmethane-4,4'-diisocyanate and polymethylene polyphenylisocyanate.

Crude polyisocyanates may also be used in the practice of the present invention, such as crude toluene diisocyanate obtained by the phosgenation of a mixture of toluenediamines or crude diphenylmethylene diisocyanate obtained by the phosgenation of crude diphenylmethylenediamine. The preferred undistilled or crude isocyanates are disclosed in US—A—3,215,652.

Of particular interest are RIM formulations which contain chain extenders, especially reactive polyamines such as ethylenediamine, 1-(2-aminoisopropyl-4-methyl-4-aminocyclohexane), 2,4-diaminotoluene, 2,6-diaminotoluene, methylene di(phenylamine), 1,2-propanediamine, 1,6-hexanediamine, diethyltoluenediamine, piperazine, N-(2-aminoethyl)-piperazine and 1,4-bis(aminomethyl)cyclohexane used in an amount effective to increase the modulus of the resultant polyurea or polyurea-urethane.

The urethane or urea reaction of TBCN diisocyanate I with one or a mixture of two or more of the aforementioned polyahls is advantageously practiced in the presence of an amount of urethane catalyst which is effective to catalyze the reaction of the polyahl with the polyisocyanate. In the case of a reaction to form a urea linkage, a catalyst is not normally required. Preferably in the formation of the urethanes, the amount of urethane catalyst is an amount comparable to that used in conventional urethane type reactions, e.g., from 0.05 to 5, most preferably from 0.1 to 3, weight percent of the catalyst based on the weight of polyahl.

Any suitable urethane catalyst may be used including tertiary amines such as, for example, triethylenediamine, N-methyl morpholine, N-ethyl morpholine, diethyl ethanolamine, N-coco morpholine, 1-methyl-4-dimethylaminoethyl piperazine, 3-methoxy-N-dimethylpropylamine, N,N-dimethyl-N',N'-methyl isopropyl propylenediamine, N,N-diethyl-3-diethylaminopropylamine, or dimethyl benzylamine. Other suitable catalysts are, for example, tin compounds such as stannous chloride, tin salts of carboxylic acids such as dibutyltin di-2-ethyl hexanoate and dibutyltin dilaurate, as well as other organometallic compounds such as are disclosed in US—A—2,846,408.

The relative proportions of the TCBN diisocyanate I to polyahl are those conventionally employed in the preparation of polyurethanes, polyureas and polyurea urethanes, preferably in proportions sufficient to provide isocyanate to active hydrogen equivalent ratios in the range from 0.8:1 to 1.5:1, most preferably from 0.95:1 to 1.1:1. The proportion of the TBCN diisocyanate I employed is that which is sufficient to improve mechanical and/or thermal properties of the polyurethane. Preferably, it is used in an amount sufficient to improve the light stability, tensile strength, optical properties or any combination of such properties for the resulting polyurethane. In the case of a polyurea or a polyurea urethane, the TBCN diisocyanate I is employed in an amount sufficient to improve light stability, resistance to heat sag, elongation, impact strength processability or any combination thereof. More preferably, the amount of TBCN diisocyanates I is in the range from 25 to 100, most preferably from 50 to 100, weight percent of the TBCN diisocyanate I based on the weight of total polyisocyanate employed.

In addition to the foregoing components, it is understood that the polyurethane formulations of the present invention may also contain suitable amounts of conventional additives such as blowing agents, fillers, surfactants and other additives such as are described in US—A—4,269,945.

Other polymers may be made by reaction of the TBCN diisocyanates I with diacids, amino-alcohols and dimercaptans.

The polymers formed with the above-described monomer units have utility as coatings, films and castings. Surprisingly, such polymers exhibit properties superior to those in which aromatic groups have been incorporated into the polymer backbone.

The following examples are given to illustrate the present invention and are not to be construed as limiting the scopes thereof in any manner. All parts and percentages are by weight unless otherwise indicated.

### Example 1a

Preparation of Dimethyl 1,5-Cyclooctadiene (III)

20 Ml (100 mmoles) of 50 percent piperylene in other $C_5$ hydrocarbons, 10 ml of toluene, 0.19 g (0.5 mmole) of tri(2,4-pentanedionato-O,O')-iron (also known as iron tri(acetylacetonate), CA registry number [14024—18—1]) and 0.3 g (1.0 mmole) of N,N'-(1,2-dimethyl-1,2-ethanediylidene)bis[4-methoxy-benzenamine] (CA registry [19215—52—2]) were combined under nitrogen in a 50-ml three-necked flask equipped with reflux condenser and nitrogen inlet. The mixture was stirred magnetically and heated to 55°C. 1.5 ml of triisobutyl aluminum (1.0M) in toluene — 1.5 mmoles) was added. A sample was taken after 1.6 hours and analyzed by gas chromatography. Piperylene was 90 percent converted. The dimethyl cyclooctadiene yield was 71 percent based on converted piperylene. In this embodiment R was methyl, R' was hydrogen.

### Example 1b

Preparation of Dimethyl TBCN Dichloride

In this embodiment, R was methyl and R' is hydrogen. The cyclooctadiene of Example 1a may be used as the starting material. This example describes a continous process setupt. The reactor used was a static inline mixer which was jacketed. A 50:50 by volume mixture of ethylene glycol and water at −30°C was circulated through the jacket. Separate inlets were provided ahead of the reaction zone for cyclooctadiene and sulfur dichloride reactant solutions. An outlet valve was provided after the reaction zone to remove products.

850 ml of dimethyl-1,5-cyclooctadiene (730 g, 5.36 moles DMCOD) containing about 12 percent other piperylene dimers was mixed with 175 ml of methylene chloride. 355 ml of sulfur dichloride (575 g, 5.58 moles) was dissolved in 3600 ml of methylene chloride.

Both reactants were fed to the reactor simultaneously. The DMCOD solution was fed at 45 ml/min. The sulfur dichloride solution was fed at 172 ml/min. The feeds are exhausted in 23 minutes. During the reaction time the temperature inside the reaction zone rose 11°C. Products were removed from the reaction zone at the same rate that they were added. When the feeds were exhausted, the reactor zone was flushed with methylene chloride. The methylene chloride was evaporated from the product to give a black oil. Distillation of this oiil at 85°C—95°C and 0.05—0.1 mm Hg (6.7—13.3 Pa) gave 1100 g of a very light yellow oil. Infrared and nuclear magnetic resonance spectra are consistent with a mixture of dimethyl TBCN dichloride isomers.

### Example 1c

Preparation of Dimethyl TBCN Diisocyanate

A 5000-ml three-necked flask equipped with nitrogen inlet/outlet, reflux condenser, thermometer, and

mechanical stirrer was dried at 150°C for 1 hour and flushed with nitrogen. To this flask was charged 382.8 g of 97.8 percent potassium cyanate (4.612 moles), 538.23 g of distilled dimethyl-dichloro-9-thiabicyclononanes (2.25 moles) and 3000 ml of acetonitrile. This mixture was stirred and heated to reflux for 3 hours. A sample taken after 3 hours showed a strong absorption for isocyanate by infrared spectroscopy (2250 $cm^{-1}$). Nuclear magnetic resonance spectroscopy indicated the reaction was complete. The reaction mixture was filtered, rotary evaporated and the residue extracted with methylene chloride. After filtration and rotary evaporation, a light yellow oil was obtained which was distilled at about 130°C and 0.10 mm Hg (13.3 Pa) to give 470 g of a colorless oil. This oil contained 33.24 percent NCO (theory, 33.30 percent) as determined by ASTM Method D—2572.

## Examples 2, 3 and 4 and Comparative Runs A and B

Polyurethane Preparation

Into the polyol side tank of a reaction injection molding machine were added a mixture of 6000 g (1.25 moles) of a glycerin-initiated polyalkylene polyol made from propylene oxide (PO) with sufficient ethylene oxide (EO) terminal groups (caps) to yield 80 percent of primary hydroxyl based on the total number of hydroxyl and having a weight ratio of PO to EO of 82:18 and a weight average molecular weight ($M_w$) between 4800 and 5000 (Polyol I), 1967 g (21.83 moles) of 1,4-butanediol, and 142 g (0.23 mole) of dibutyltin dilaurate (urethane catalyst). These ingredients were mechanically agitated and heated to 100°F (37.8°C). To the isocyanate tank of the reaction injection molding machine was added 6271 g (24.28 moles) of a normally liquid mixture of isomers of dimethyl-9-thiabicyclononane diisocyanate and then agitated and heated to 100°F (37.8°C). Specific gravities of the ingredients in each side tank of the reaction injection molding machine were 1.22 g/ml in the isocyanate tank and 1.04 g/ml in the polyol tank. The mixing pressure used for impingement was 1500 psi (10.34 MPa) and the ingredients were shot into the mold using approximately a 0.65 lb (0.29 kg) shot size and a 40 lb/min (18 kg/min) throughput. The temperature of the mold was 155°F (68.3°C) and the in-mold time for each shot of material was 2 minutes. The dimensions of the mold cavity were 254 × 254 × 3.2 mm. Upon removal from the mold, the resulting molded article was placed in an oven at 120°C for a post-cure of 30 or 90 minutes as indicated in the following Table II. The molded material was then tested for physical properties.

For Examples 2 and 3, formulations were prepared using different concentrations of the same or different ingredients such as the diisocyanate, polyether polyol, diol, polyamine chain extender and the catalyst as specified in Table I. The resultant molded articles were similarly tested for physical properties and the results are reported in the following Table II.

For Comparative Runs A and B, conventional formulations were prepared using the foregoing ingredients except that diphenylmethane diisocyanate (MDI) or isophorone diisocyanate (IPDI) is substituted for the thiapolycyclic diisocyanate of the present invention. These Comparative Runs were similarly processed and tested for physical properties and the results are reported in Table II.

TABLE I

| Example or Comp. Run | Diisocyanate[1] | | | Polyether Polyol[2] | | Diol[3] | | | Amine Extender[4] | | | Catalyst[5] |
|---|---|---|---|---|---|---|---|---|---|---|---|---|
| | Type | % | Equiv | % | Equiv | Type | % | Equiv | Type | % | Equiv | % |
| 2 | DMTBNI | 43.6 | 49.77 | 41.7(I) | 3.75 | BD | 13.68 | 43.65 | — | — | — | 1.0[a] |
| 3 | DMTBNI | 43.9 | 50.56 | 41.4(I) | 3.75 | BD | 13.70 | 44.12 | — | — | — | 1.0[a] |
| 4 | DMTBNI | 38.5 | 23.68 | 47.0(II) | 2.43 | BD | 7.0 | 13.27 | DETDA | 7.1 | 6.78 | 0.4[b] |
| A | MDI | 47.5 | 50.20 | 39.46(I) | 3.75 | BD | 12.91 | 43.56 | — | — | — | 0.1[a] |
| B | IPDI | 33.4 | 23.66 | 50.9(II) | 2.43 | BD | 7.6 | 13.34 | DETDA | 7.6 | 6.74 | 0.4[b] |

[1]DMTBNI — dimethyl-9-thiabicyclononane diisocyanate
IPDI — isophorone diisocyanate
MDI — diphenylmethane diisocyanate
% based on weight of total urethane formulation, equivalent of isocyanate functionality
[2](I) — Polyol I as defined
(II) — a PO/EO polyol (75% primary hydroxyl) wherein 30 mole percent of hydroxyl groups are substituted with primary amine groups.
% based on total urethane formulation, equivalents of hydroxyl functionality (80% primary hydroxyl)
[3]BD — 1,4-butane diol
% based on total urethane formulation, equivalents of hydroxyl functionality
[4]DETDA — diethyltoluenediamine
% based on total urethane formulation, equivalents of amine functionality
[5a]dibutyltin dilaurate
[b] mixture of (1) dimethyltin carboxylate (2 parts)
(2) 33% of triethylendiamine in dipropylene glycol (1 part)
(3) dimethyltin dilaurate (1 part)
% based on urethane formulation

TABLE II

| Example or Comp. Run | Time min. | Tensile[1] Strength psi (MPa) | Tensile[1] Modulus psi (MPa) | % Elongation | Flexural[2] Strength psi (MPa) | Flexural[2] Modulus psi (MPa) | Izod[2] ft-lb/min (Joules/mm) |
|---|---|---|---|---|---|---|---|
| 2 | 90 | 2945 (20.3) | — | 180 | — | — | — |
| 3 | 90 | 2598 (19.9) | — | 186 | — | — | — |
| 4 | 90 | 2240 (15.4) | 38,000 (262) | 180 | 1381 (9.52) | 30,000 (207) | 6.79 (0.363) |
| A | 90 | 1958 (13.5) | — | 60 | — | — | — |
| B | 30 | 1600 (11.0) | 14,000 (96.5) | 480 | 716 (4.94) | 20,000 (138) | — |

[1]ASTM D 638
[2]ASTM D 790
[3]ASTM D 265

As evidenced by the data shown in Tables I and II, the polyurethanes prepared by the practice of the present invention (Examples 2, 3 and 4) exhibit better tensile and flexural properties than do polyurethanes using conventional diisocyanates.

Examples 5, 6 and 7 and Comparative Run C

Into the polyol side tank of a reaction injection molding machine (Accuratio VR—60 RIM Machine) were added 6,743 g (1.35 moles) of a trifunctional polyether polyol having a $M_w$ of 5000 capped with approximately 87 percent with primary amine (sold by Texaco under the trade name ®Jeffamine T—5000), 1,212 g (6.8 moles) of diethyltoluenediamine, 243 g (4.1 mols) of ethylene dimaine and 13.5 g of a delayed action urethane catalyst. These ingredients were mechanically agitated and heated to 140°F (60°C). To the isocyanate tank of the reaction injection molding machine was added 3,673 g (13.32 moles) of a normally liquid mixture of isomers of dimethyl-9-thiabicyclononane diisocycante and then agitated and heated to 140°F (60°C). Specific gravities of the ingredients in each side tank of the reaction injection molding machine were 1.22 g/ml in the isocyanate tank and 1.04 g/ml in the polyol tank. The mixing pressure used for impingement was 2000 psi (13.8 MPa) and the ingredients were shot into the mold using approximately a 0.65 lb (0.29 kg) shot size and a 40 lb/min (18 kg/min) throughout. The temperature of the mold was 160°F (71.1°C) and the in-mold time for each shot of material was 2 minutes. The dimensions of the mold cavity were 254 × 254 × 3.2 mm. Upon removal from the mold, the resulting molded article was placed in an oven at 150°C for a post-cure of 30 or 60 minutes as indicated in the following Table III. The molded material was then tested for physical properties.

For Examples 6 and 7, formulations were prepared using different concentrations of various ingredients such as the diisocyanate, the aminated polyol, amine chain extenders and the catalyst as specified in Table III. The resultant molded articles were smilarly tested for physical properties and the results are reported in Table IV.

For Comparative Run C, a conventional formulation was prepared using the foregoing ingredients except that diphenylmethane diisocycante was substituted for the thiapolycyclic diisocyanate. This formulation was similarly molded and the results are reported in Table IV.

TABLE III

| Ex or Comp Run | Diisocyanate[1] | | | Polyol[2] | | | DETDA[3] | | Other Diamine[4] | | | Catalyst[5] | |
|---|---|---|---|---|---|---|---|---|---|---|---|---|---|
| | Type | % | Equiv | Type | % | Equiv | % | Eqiv | Type | % | Eqiv | Type | % |
| 5 | DMTBNI | 30.9 | 26.62 | T—5000 | 56.7 | 4.04 | 10.2 | 13.62 | EDA | 2.0 | 8.1 | UL—29 | 0.1 |
| 6 | DMTBNI | 26.2 | 15.46 | T—5000 | 61.5 | 3.00 | — | — | DAM | 12.3 | 11.72 | — | — |
| 7 | DMTBNI | 27.9 | 20.03 | T—5000 | 65.6 | 3.90 | — | — | PIP | 6.6 | 15.17 | — | — |
| C | MDI | 57.21 | 73.95 | P—I | 29.85 | 3.50 | — | — | D—400 | 2.09 | 1.958 | T—12 | 0.003 |
| | | | | EG | 10.76 | 64.97 | — | — | | | | UL—28 | 0.06 |

[1]Same as [1] in Table I

[2]T—5000 — ®Jeffamine T—5000 sold by Texaco; P—I — Polyol I, same as defined in Example 1;
EG — ethylene glycol
% based on weight of total polyurea formulation; equivalent of total amine and hydroxyl

[3]Diethyltoluene diamine
% based on total polyurea formulation; equivalent of amine functionality

[4]EDA — ethylene diamine; DAM — 1-(2-aminoisopropyl-4-methyl-4-aminocyclohexane; PIP — piperazine; D—400 — amine terminated polyol which is a diamine having an average of 5.6 propyleneoxy units per molecule and sold by Texaco under the trade name ®Jeffamine D—400
% based on total polyurea formulation; equivalent of amine functionality

[5]UL—29 — delayed action urethane catalyst; UL—28 — dimethyltin dilaurate; T—12 — dibutyltin dilaurate
% based on total polurea formulation.

TABLE IV

| Ex. or Comp. Run | Time min. | Tensile[1] Strength psi (MPa) | Tensile[1] Modulus psi (MPa) | % Elongation | Flexural[2] Strength psi (MPa) | Flexural[2] Modulus psi (MPa) | Izod[3] ft-lb/in (Joules/mm) | Heat Sag[4] in (mm) 6 in (152 mm) |
|---|---|---|---|---|---|---|---|---|
| 6 | 60 | 1360 (9.38) | 43,000 (296) | 326 | 1090 (7.51) | 50,000 (345) | 9.64 (0.515) | 0.22 (5.6) |
| C | 30 | 3712 (25.6) | 80,000 (552) | — | 3790 (26.1) | 100,000 (689) | 7.27 (0.388) | 0.79 (20) |

[1]ASTM D 638
[2]ASTM D 790
[3]ASTM D 265
[4]ASTM D 376—81 (30 min. at 325°F (163°C))

As shown by the data in Tables III and IV, the polyureas prepared by the practice of the present invention (Example 6) exhibited better heat sag properties than the polyurethane control sample (Comparative Run C).

**Claims**

1. A 9-thiabicyclononanediisocyanate of the structural formula:

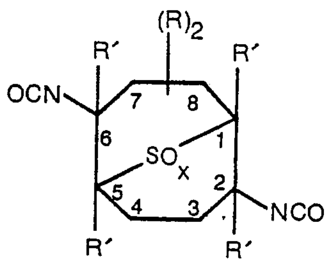

or

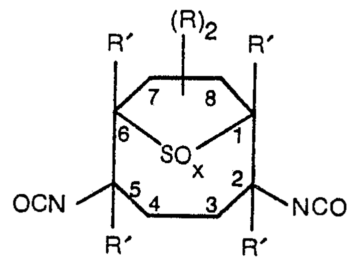

(I)

wherein

each R, which may be the same or different, is an alkyl group containing 1 to 3 carbon atoms;

each R′, which may be the same or different, is hydrogen or methyl, at least two R′ are hydrogen; and x is 0, 1 or 2.

2. The diisocyanate of Claim 1 wherein all R′ are hydrogen.

3. The diisocyanate of Claim 2 wherein x is zero.

4. The diisocyanate of Claim 3 wherein each R is methyl.

5. The diisocyanate of Claim 4 wherein the diisocyanate is a liquid at room temperature.

6. A polyurethane, polyurea, or polyurea-urethane characterized by containing monomer units having the formula:

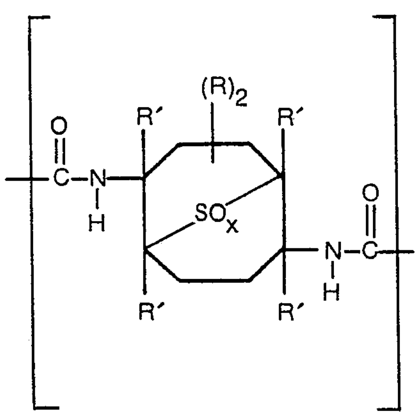

or

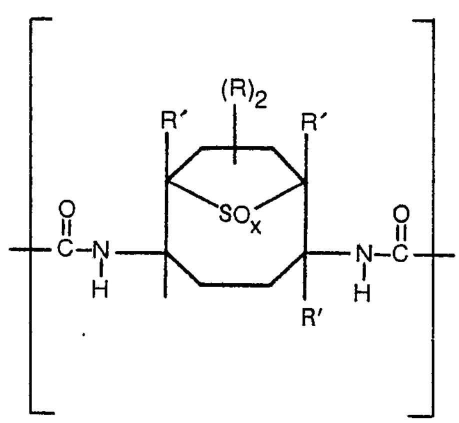

(V)

wherein

each R, which may be the same or different, is an alkyl group containing 1 to 3 carbon atoms;

each R′, which may be the same or different, is hydrogen or methyl, at least two R′ are hydrogen; and x is 0, 1 or 2.

7. The polymer of Claim 6 wherein all R′ are hydrogen.

8. The polymer of Claim 7 wherein x is zero.

9. The polymer of Claim 8 wherein each R is methyl.

10. The polymer of Claim 6 wherein at least about 0.5 mole percent of the monomer units is present.

**Patentansprüche**

1. 9-Thiabicyclononan/diisocyanat der Strukturformel:

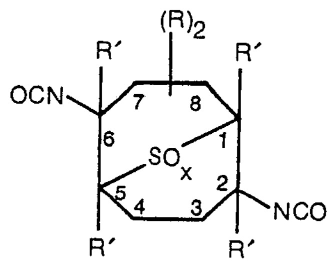

oder

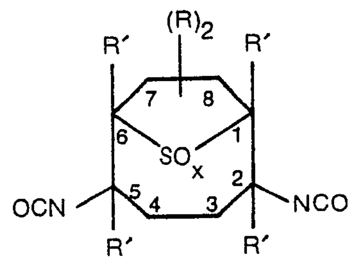

(I)

in der jedes R, das gleich oder unterschiedlich sein kann, eine Alkylgruppe mit 1 bis 3 Kohlenstoffatomen ist, jedes R′, das gleich oder unterschiedlich sein kann, Wasserstoff oder Methyl ist, mindestens zwei R′ Wasserstoff sind und X 0, 1 oder 2 ist.

2. Diisocyanat nach Anspruch 1, dadurch gekennzeichnet, daß alle R′ Wasserstoff sind.

3. Diisocyanat nach Anspruch 2, dadurch gekennzeichnet, daß X Null ist.

4. Diisocyanat nach Anspruch 3, dadurch gekennzeichnet, daß jedes R Methyl ist.

5. Diisocyanat nach Anspruch 4, dadurch gekennzeichnet, daß das Diisocyanat bei Raumtemperatur eine Flüssigkeit ist.

6. Polyurethan, Polyharnstoff oder Polyharnstoff-Urethan, gekennzeichnet durch enthaltend Monomereinheiten der Formel

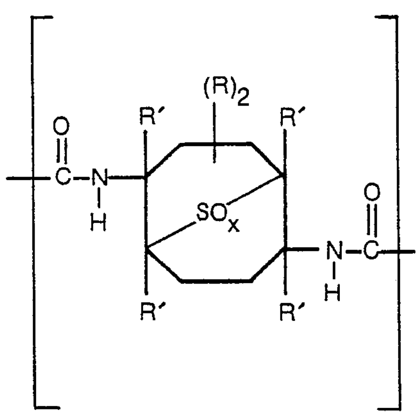

oder

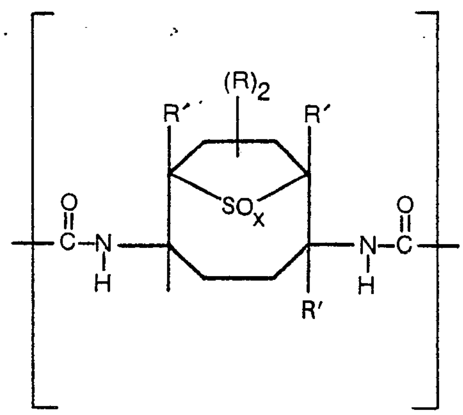

(V)

in der jedes R, das gleich oder unterschiedlich sein kann, eine Alkylgruppe mit 1 bis 3 Kohlenstoffatomen ist, jedes R′, das gleich oder unterschiedlich sein kann, Wasserstoff oder Methyl ist, mindestens zwei R′ Wasserstoff sind X 0, 1 oder 2 ist.

7. Polymer nach Anspruch 6, dadurch gekennzeichnet, daß alle R′ Wasserstoff sind.

8. Polymer nach Anspruch 7, dadurch gekennzeichnet, X Null ist.

9. Polymer nach Anspruch 8, dadurch gekennzeichnet, daß jedes R Methyl ist.

10. Polymer nach Anspruch 6, dadurch gekennzeichnet, daß mindestens 0.5 Molprozent der Monomereinheiten anwesend sind.

**Revendications**

1. 9-thiabicyclononanediisocyanate de formule structurelle:

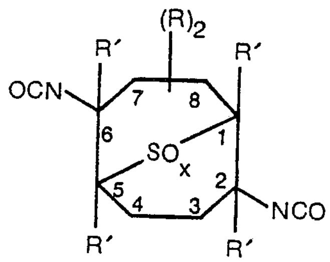

ou

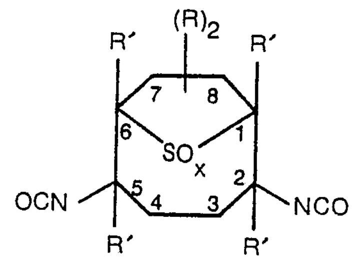

(I)

dans laquelle chacun des R, qui peuvent être identiques ou différents, est un groupe alkyle contenant de 1 à 3 atomes de carbone; chacun des R', qui peuvent être identiques ou différents, est un atome hydrogène ou un méthyle, au moins deux R' étant des atomes d'hydrogène; et x vaut 0, 1 ou 2.

2. Diisocyanate selon la revendication 1, dans lequel tous les R' sont des atomes d'hydrogène.

3. Diisocyanate selon la revendication 2, dans lequel x vaut 0.

4. Diisocyanate selon la revendication 3, dans lequel chaque R est un méthyle.

5. Diisocyanate selon la revendication 4, dans lequel le diisocyanate est un liquide à la température ambiante.

6. Polyuréthane, polyurée ou polyurée-uréthane, caractérisé en ce qu'il contient des motifs monomères ayant la formule:

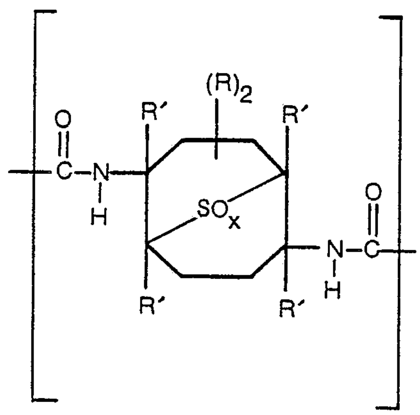

ou

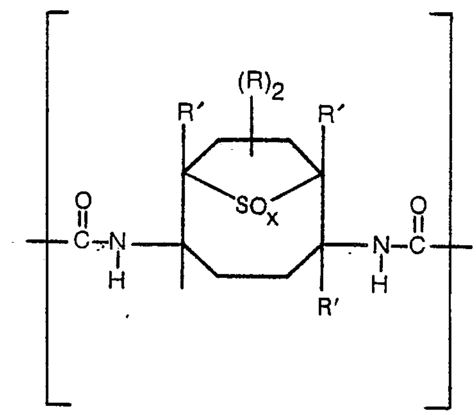

(V)

dans laquelle chacun des R, qui peuvent être identique ou différents, est un groupe alkyle contenant de 1 à 3 atomes de carbone; chacun des R', qui peuvent être indentiques ou différents, est un atome d'hydrogène ou un méthyle, au moins deux R' étant des atomes d'hydrogène; et x vaut 0, 1 ou 2.

7. Polymère selon la revendication 6, dans lequel tous les R sont des atomes d'hydrogène.

8. Polymère selon la revendication 7, dans lequel x vaut 0.

9. Polymère selon la revendication 8, dans lequel chaque R est un méthyle.

10. Polymère selon la revendication 6, dans lequel il y a au moins environ 0.5% en moles des motifs monomères.